# EUROPÄISCHE PATENTSCHRIFT

(11) **EP 1 412 613 B1**
(45) Veröffentlichungstag und Bekanntmachung des Hinweises auf die Patenterteilung: **26.01.2005**
(21) Anmeldenummer: 02754766.0
(22) Anmeldetag: 27.06.2002
(51) Int. Cl.: E21B 37/06, C10L 3/00, C07C 219/06, E21B 41/02

(54) **ADDITIVE ZUR INHIBIERUNG DER GASHYDRATBILDUNG**
ADDITIVES FOR INHIBITING THE FORMATION OF GAS HYDRATES
ADDITIFS POUR INHIBER LA FORMATION D'HYDRATE DE GAZ

(30) Priorität: 13.07.2001 DE 10134224
(43) Veröffentlichungstag der Anmeldung: 28.04.2004
(73) Patentinhaber: Clariant GmbH, 65929 Frankfurt am Main (DE)
(72) Erfinder: DAHLMANN, Uwe, 69126 Heidelberg (DE); FEUSTEL, Michael, 55278 Köngernheim (DE)
(74) Vertreter: Mikulecky, Klaus
(86) Internationale Anmeldenummer: PCT/EP2002/007096
(87) Internationale Veröffentlichungsnummer: WO 2003/008757

(56) Entgegenhaltungen:
- WO-A-96/34177
- WO-A-99/13197

## Beschreibung

Die vorliegende Erfindung betrifft ein Additiv, seine Verwendung und ein Verfahren zur Inhibierung von Keimbildung, Wachstum und/oder Agglomeration von Gashydraten, indem einem zur Hydratbildung neigenden, aus Wasser, Gas und ggf. Kondensat bestehenden Mehrphasengemisch oder einer zur Gashydratbildung neigenden Bohrspülflüssigkeit eine wirksame Menge eines Inhibitors zugegeben wird, der zweifach alkoxylierte quartäre Ammoniumverbindungen enthält.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25°C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck- und Temperaturverhältnissen zu Problemen führen, indem sich in den Bohrspülflüssigkeiten Gashydrate bilden.

Um solche Probleme zu vermeiden, kann die Gashydraibildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Anti-Agglomerates). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Agglomeratinhibitoren darstellen. Von besonderer Bedeutung sind hierbei Polymere mit Kohlenstoff-Backbone, die in den Seitengruppen sowohl cyclische (Pyrrolidon- oder Caprolactamreste) als auch acyclische Amidstrukturen enthalten.

EP-B-0 736 130 offenbart ein Verfahren zur Inhibierung von Gashydraten, welches die Zuführung einer Substanz der Formel 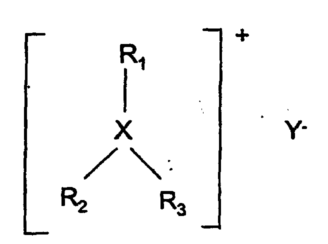 mit X = S, N - R₄ oder P - R₄, R₁, R₂ und R₃ = Alkyl mit mindestens 4 kohlenstoffatomen, R₄ = H oder ein organischer Rest, und Y = Anion erfordert. Umfasst werden also Verbindungen der Formel 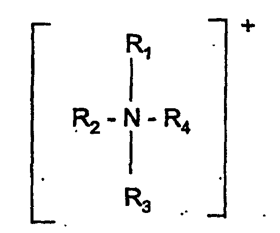 worin R₄ ein beliebiger Rest sein kann, die Reste R₁ bis R₃ aber Alkylreste mit mindestens 4 Kohlenstoffatomen darstellen müssen. Es ist keine zweifache Alkoxylierung offenbart.

EP-B-0 824 631 offenbart ein Verfahren zur Inhibierung von Gashydraten, welches die Zuführung einer Substanz der Formel 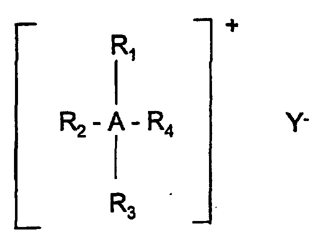 mit R₁, R₂ = gerade/verzweigte Alkylreste mit 4 oder 5 Kohlenstoffatomen, R₃, R₄ = organische Reste mit mindestens 8 Kohlenstoffatomen und A = Stickstoff oder Phosphor erfordert. Y⁻ ist ein Anion. Es müssen zwei der Reste R₁ bis R₄ gerade oder verzweigte Alkylreste mit 4 oder 5 Kohlenstoffatomen sein, eine zweifache Alkoxylierung wird nicht offenbart.

US-5 648 575 offenbart ein Verfahren zur Inhibierung von Gashydraten. Das Verfahren umfasst die Verwendung einer Verbindung der Formel 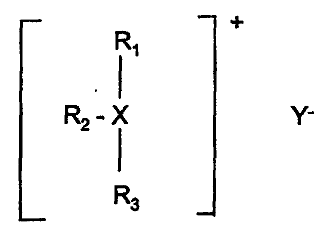 worin R¹, R² gerade oder verzweigte Alkylgruppen mit mindestens 4 Kohlenstoffatomen, R³ ein organischer Rest mit mindestens 4 Atomen, X Schwefel, NR⁴ oder PR⁴, R⁴ Wasserstoff oder ein organischer Rest, und Y ein Anion sind. Das Dokument offenbart nur solche Verbindungen, die mindestens zwei Alkylreste mit mindestens 4 Kohlenstoffatomen aufweisen, eine zweifache Alkoxylierung wird nicht offenbart.

US-6 025 302 offenbart Polyetheraminammoniumverbindungen als Gashydratinhibitoren, deren Ammoniumstickstoffatom neben der Polyetheraminkette 3 Alkylsubstituenten trägt.

US-5 523 433 offenbart Verbindungen der Formel 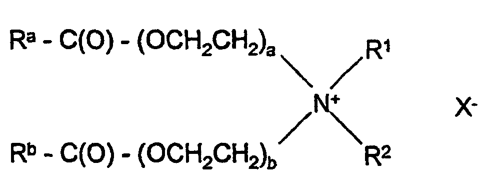 worin R^{a} und R^{b} für C₁₂- bis C₂₂-Alkylreste und R¹ und R² für C₁- bis C₄-Alkylreste stehen können. Das Dokument offenbart die Eignung solcher Verbindungen als Bestandteil von Wäscheweichspülern.

WO-99/13197 offenbart Ammoniumverbindungen als Gashydratinhibitoren, die auch alkoxyliert sein können, aber nicht die Vorteile einer zweifachen N-Alkoxylierung.

WO-01/09082 offenbart quartäre Amide als Gashydratinhibitoren, die jedoch keine Alkoxygruppen tragen.

WO-00/078 706 offenbart quartäre Ammoniumverbindungen als Gashydratinhibitoren, die jedoch keine Carbonylreste tragen.

Die beschriebenen Additive besitzen nur eingeschränkte Wirksamkeit als kinetische Gashydratinhibitoren und/oder Anti-Agglomerates, müssen mit Co-Additiven verwendet werden, oder sind nicht in ausreichender Menge oder nur zu hohen Preisen erhältlich.

Um Gashydratinhibitoren auch bei stärkerer Unterkühlung als zur Zeit möglich, d.h. weiter innerhalb der Hydratregion einsetzen zu können, bedarf es einer weiteren Wirkungssteigerung im Vergleich zu den Hydratinhibitoren des Standes der Technik. Zusätzlich sind in Bezug auf ihre biologische Abbaubarkeit, antikorrosiven Eigenschaften und Toxizität verbesserte Produkte erwünscht.

Aufgabe der vorliegenden Erfindung war es also, verbesserte Additive zu finden, die sowohl die Bildung von Gashydraten verlangsamen (kinetische Inhibitoren) als auch Gashydratagglomerate klein und pumpbar halten (Anti-Agglomerates), um so ein breites Anwendungsspektrum mit hohem Wirkpotential zu gewährleisten. Des weiteren sollten die derzeit verwendeten thermodynamischen Inhibitoren (Methanol und Glykole), die beträchtliche Sicherheitsprobleme und Logistikprobleme verursachen, ersetzt werden können.

Gashydratinhibitoren des Standes der Technik werden gewöhnlich mit Korrosionsinhibitoren co-additiviert, um Korrosion der Transport- und Fördereinrichtungen vorzubeugen. Aufgrund der häufig nicht unmittelbar gegebenen Verträglichkeit von Gashydratinhibitor und Korrosionsschutzmittel bei der Formierung ergibt sich daraus für den Anwender ein zusätzlichen Aufwand. Ein wesentlicher Vorteil gegenüber dem Stand der Technik bestände, wenn eine Co-Additivierung mit Korrosionsinhibitoren nicht mehr zwingend erforderlich ist.

Es wurde nun überraschend gefunden, dass zweifach N- alkoxylierte und carbonylierte Ammoniumsalze ausgezeichnete Wirkung als Gashydratinhibitoren aufweisen. Ihre korrosionsinhibierende Wirkung ist so gut, dass keine Additivierung mit weiteren Korrosionsinhibitoren erforderlich ist.

Gegenstand der Erfindung ist somit die Verwendung von Verbindungen der Formel 1 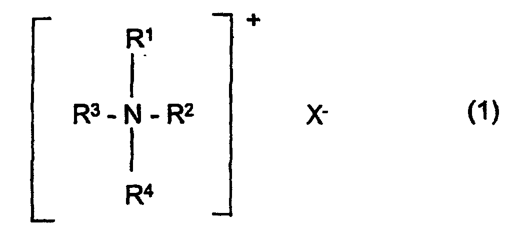 worin
- R¹, R²: unabhängig voneinander Reste der Formeln

-(B)-(O-A)ₙ-O-CO-R⁵ (2)

oder

-(A-O)ₙ-(C)-CO-O-R⁵ (3)
- R³: C₁- bis C₃₀-Alkyl oder C₂- bis C₃₀-Alkenyl
- R⁴: einen gegebenenfalls Heteroatome enthaltenden organischen Rest mit 1 bis 100 Kohlenstoffatomen
- R⁵: C₁- bis C₃₀-Alkyl oder C₂- bis C₃₀-Alkenyl
- n: eine Zahl von 1 bis 20
- A: eine C₂- bis C₄-Alkylengruppe,
- B: eine C₁- bis C₁₀-Alkylengruppe,
- C: eine C₁- bis C₆-Alkylengruppe und
- X: ein Anion
bedeuten, als Gashydratinhibitoren.

Ein weiterer Gegenstand der Erfindung ist ein Verfahren zur Inhibierung von Gashydraten, indem einem zur Bildung von Gashydraten neigenden System aus Wasser und Kohlenwasserstoffen mindestens eine Verbindung der Formel 1 zugesetzt wird.

Ein weiterer Gegenstand der Erfindung sind die Verbindungen der Formel (1), wobei jedoch solche Verbindungen ausgeschlossen sind, in denen R⁴ kein Heteroatom enthält und R¹ und R² gleichzeitig die in Formel (2) angegebene Bedeutung aufweisen.

Kohlenwasserstoffe im Sinne dieser Erfindung sind leichtflüchtige Kohlenwasserstoffe, wie beispielsweise Methan, Ethan, Propan, Butan. Für die Zwecke dieser Erfindung zählen dazu auch die weiteren gasförmigen Bestandteile des Erdöls/Erdgases, wie etwa Schwefelwasserstoff und Kohlendioxid.

A kann geradkettig oder verzweigt sein und steht vorzugsweise für eine Ethylen- oder Propylengruppe, insbesondere eine Ethylengruppe. Bei den durch (A-O)ₙ bezeichneten Alkoxygruppen kann es sich auch um gemischte Alkoxygruppen handeln.

B kann geradkettig oder verzweigt sein und steht vorzugsweise für eine C₂- bis C₄-Alkylengruppe, insbesondere für eine Ethylen- oder Propylengruppe.

C kann geradkettig oder verzweigt sein und steht vorzugsweise für eine C₂- bis C₄-Alkylengruppe, insbesondere für eine Methylen- oder Ethylengruppe.

n steht vorzugsweise für eine Zahl zwischen 2 und 6.

R⁵ steht vorzugsweise für eine Alkyl- oder Alkenylgruppe mit 2 bis 24 Kohlenstoffatomen, insbesondere 4 bis 12 Kohlenstoffatomen.

R³ steht vorzugsweise für eine Alkyl- oder Alkenylgruppe von 2 bis 12 Kohlenstoffatomen, insbesondere für solche Gruppen mit 4 bis 8 Kohlenstoffatomen und speziell für Butyl-Gruppen.

R⁴ kann ein beliebiger organischer Rest sein, der 1 bis 100 C-Atome enthält, und der Heteroatome enthalten kann. Enthält R⁴ Heteroatome, so handelt es sich vorzugsweise um Stickstoff- oder Sauerstoffatome oder beides, vorzugsweise um beides. Die Stickstoffatome können in quatemierter Form vorliegen.

In einer weiteren bevorzugten Ausführungsform umfasst R⁴ 1 bis 20 Alkoxygruppen, die von C₂- bis C₄-Alkylenoxid abgeleitet sind, insbesondere von Ethylenoxid und/oder Propylenoxid. Insbesondere kann R⁴ für einen Rest nach Formel (2) oder (3) stehen.

In einer besonders bevorzugten Ausführungsform entspricht R⁴ einem Rest der Formel (4) 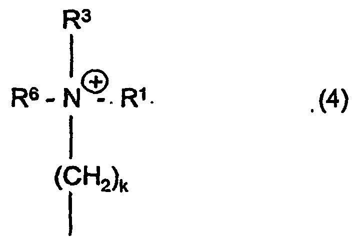 wobei die Bindung an das Stickstoffatom in Formel 1 über die freie Valenz der (CH₂)ₖ-Gruppe erfolgt. In Formel (4) bedeutet R⁶ einen Rest der Formeln

-(B)-(O-A)ₙ-O-CO-R⁵ (2)

oder

-(A-O)ₙ-(C)-CO-O-R⁵ (3)

oder C₁- bis C₃₀-Alkyl oder C₂- bis C₃₀-Alkenyl, jeweils mit den weiter oben für A, B, n, R³ und R⁵ angegebenen Vorzugsbereichen. k steht für 2 oder 3, R¹ und R³ haben die oben angegebenen Bedeutungen.

Als Gegenionen X sind alle Ionen geeignet, die die Löslichkeit der Verbindungen der Formel (1) in den zur Gashydratbildung neigenden organisch-wässrigen Mischphasen nicht beeinträchtigen. Solche Gegenionen sind beispielsweise Methylsulfationen (Methosulfat) oder Halogenidionen.

Besonders bevorzugte Verbindungen (ohne Gegenionen dargestellt) entsprechen den Formeln (5) bis (8)

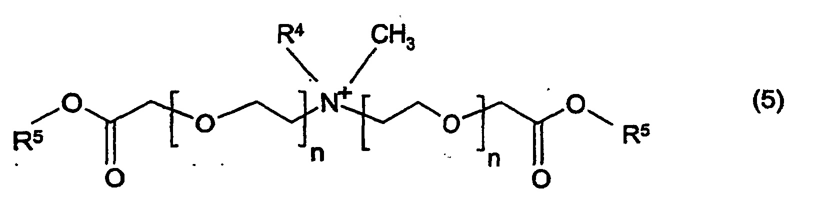

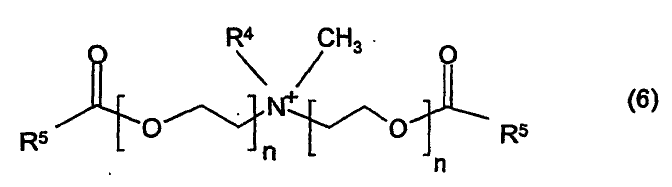

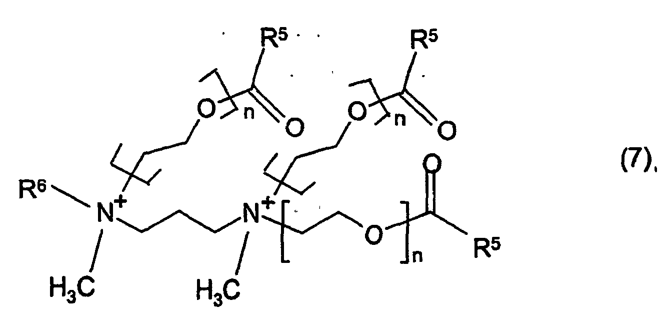

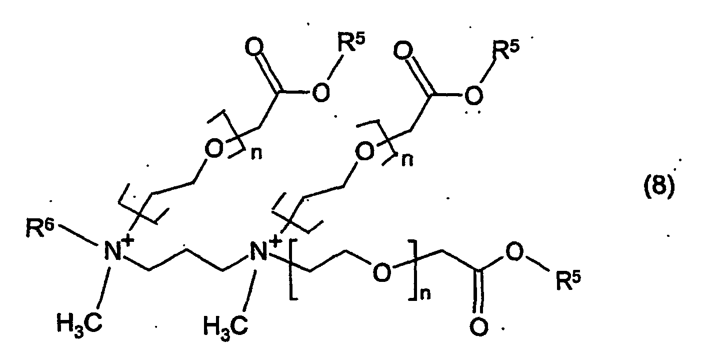

Die erfindungsgemäßen Verbindungen können alleine oder in Kombination mit anderen bekannten Gashydratinhibitoren eingesetzt werden. Im allgemeinen wird man dem zur Hydratbildung neigenden System soviel des erfindungsgemäßen Gashydratinhibitors zusetzen, dass man unter den gegebenen Druck- und Temperaturbedingungen eine ausreichende Inhibierung erhält. Die erfindungsgemäßen Gashydratinhibitoren werden im allgemeinen in Mengen zwischen 0,01 und 5 Gew.-% (bezogen auf das Gewicht der wässrigen Phase), entsprechend 100 - 50.000 ppm, vorzugsweise 0,02 bis 1 Gew.-% verwendet. Werden die erfindungsgemäßen Gashydratinhibitoren in Mischung mit anderen Gashydratinhibitoren verwendet, so beträgt die Konzentration der Mischung 0,01 bis 2 bzw. 0,02 bis 1 Gew.-% in der wässrigen Phase.

Die erfindungsgemäßen Verbindungen werden vorzugsweise zur Anwendung als Gashydratinhibitoren in alkoholischen Lösungsmitteln wie wasserlösliche Monoalkohole, z.B. Methanol, Ethanol, Propanol, Butanol, sowie oxethylierten Monoalkoholen, wie Butylglykol, Isobutylglykol, Butyldiglykol und Polyglykole gelöst.
Desweiteren wurde überraschend gefunden, dass die erfindungsgemäßen Verbindungen entsprechend Formel (1) und (4) als Korrosionsinhibitoren wirken. Eine zusätzliche Additivierung mit Korrosionsinhibitoren ist somit gegebenenfalls nicht mehr nötig, so dass aufwendiges Formulieren unter Berücksichtigung der Verträglichkeit von Gashydratinhibitor und Korrosionsschutzkomponente für den Anwender entfällt.

Die erfindungsgemäßen Verbindungen können dadurch hergestellt werden, dass man alkoxylierte Alkylamine bzw. Alkylaminoalkylenamine mit Monochlorcarbonsäuren zu den entsprechenden Ethercarbonsäuren umsetzt und anschließend mit Alkanolen verestert. Andererseits können die bisalkoxylierten Monoalkylamine bzw. Alkylamino- alkylenamine direkt mit Carbonsäuren und deren Derivaten, wie Anhydride, Carbonsäurechloride bzw. deren Ester, zu den erfindungsgemäßen Estern umgesetzt werden. Danach erfolgt die Quaternierung mit geeigneten Alkylierungsmitteln.

Die Herstellung von alkoxylierten Alkylaminen bzw. Alkylaminoalkylenaminen ist im Stand der Technik beschrieben.

Basis der verwendeten alkoxylierten Alkylamine sind Alkylamine mit C₁- bis C₃₀-Alkylresten oder C₂- bis C₃₀-Alkenylresten, bevorzugt C₃- bis C₈-Alkylamine. Geeignete Alkylamine sind beispielsweise n-Butylamin, Isobutylamin, Pentylamin, Hexylamin, Octylamin, Cyclopentylamin, Cyclohexylamin.

Basis der verwendeten alkoxylierten Alkylaminoalkylenamine sind Aminoalkylenamine mit C₁- bis C₃₀-Alkylresten oder C₂- bis C₃₀-Alkenylresten und k = 2 oder 3. Geeignete Aminoalkylenamine sind beispielsweise Fettalkylpropylendiamine wie Talgfettpropylendiamin, Stearylpropylendiamin, Oleylpropylendiamin, Laurylpropylendiamin, Dodecylpropylendiamin und Octylpropylendiamin.

Die Alkylamine bzw. Alkylaminoalkylenamine werden im allgemeinen mit Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf Alkylamin bzw. Alkylaminoalkylenamine werden 1-40 Mol Alkylenoxid beaufschlagt, bevorzugt 1-12 Mol.

Die Alkoxylierung erfolgt in Substanz, kann aber auch in Lösung durchgeführt werden. Geeignete Lösemittel für die Alkoxylierung sind inerte Ether wie Dioxan, Tetrahydrofuran, Glyme, Diglyme und MPEGs.

Im Allgemeinen wird die Alkoxylierung im ersten Reaktionsschritt unkatalysiert bis auf > 95 Gew.-% tert.- Stickstoff durchgeführt. Höheralkoxylierung erfolgt nach Zugabe basischer Verbindungen als Katalysatoren. Als basische Verbindungen können Erdalkali-/Alkalimetallhydroxide oder- alkoholate (Natriummethylat, Natriumethylat, Kalium-tert.-butylat) verwendet werden, bevorzugt sind aber Alkalimetallhydroxide, besonders Natriumhydroxid oder Kaliumhydroxid.

Für die Herstellung der erfindungsgemäßen Verbindungen werden in einem anschließenden Reaktionsschritt die Amin- Oxethylat- Mischungen mit einem Chlorcarbonsäurederivat und einer Base, bevorzugt trockenem Chloressigsäure-Natriumsalz und Natriumhydroxid umgesetzt. Dies kann geschehen, indem man die Oxethylat- Mischung mit 100 bis 150 Mol-% Natriumchloracetat bei 30 bis 100°C umsetzt und gleichzeitig oder nacheinander mit festem Natriumhydroxid oder Kaliumhydroxid versetzt, so dass die Summe aus der in der Oxethylat-Mischung bereits vorliegenden Base und der zusätzlich zugegebenen Basenmenge der Menge an Natriumchloracetat entspricht. Die aus der Umsetzung mit dem Alkylenoxid bereits enthaltene Basenmenge kann somit direkt für die anschließende Williamson-Synthese genutzt werden und muss nicht, wie bei der Synthese eines Standard- Oxethylats, ausgewaschen werden.

Anschließend an die Alkylierungsreaktion werden die alkoxylierten Amin-Ethercarbonsäure- Alkalisalze in die freie Ethercarbonsäure überführt. Hierzu wird mit starker Mineralsäure (Salzsäure, Schwefelsäure) auf pH < 3 angesäuert und die Ethercarbonsäure durch Phasentrennung oberhalb ihres Trübungspunktes heiß als Oberphase abgetrennt.

Die anschließende Veresterung der alkoxylierten Amin- Ethercarbonsäuren erfolgt im allgemeinen durch direkte Umsetzung der freien Säure mit entsprechenden Alkoholen bei Temperaturen von 100 - 200 °C, wobei das Reaktionswasser destillativ entfernt wird. Die Veresterung kann durch Zugabe geeigneter saurer Katalysatoren mit einem pKₐ- Wert von kleiner gleich 5 oder durch Auskreisen des Reaktionswassers mit geeigneten Lösemitteln beschleunigt werden. Geeignete Katalysatoren sind beispielsweise Sulfonsäure und Alkylstannansäuren.

Für die Veresterung der alkoxylierten Amin- Ethercarbonsäuren werden Alkohole mit C₄- bis C₃₀-Alkylresten oder C₄- bis C₃₀-Alkenylresten verwendet, bevorzugt Fettalkohole. Geeignete Alkohole stellen beispielsweise 2-Ethylhexanol, Octanol, Decanol, Laurylalkohol, Palmitylalkohol, Stearylalkohol und Oleylalkohol dar.

Die erfindungsgemäßen Verbindungen können auch durch Veresterung der Amin-Oxethylat- Mischungen mit Carbonsäuren und deren Derivaten, wie Carbonsäurechloride, Carbonsäureanhydride und Carbonsäureester hergestellt werden. Die Veresterung mit freien Carbonsäuren erfolgt bei Temperaturen von 100 - 200 °C, wobei das Reaktionswasser destillativ entfernt wird. Die Veresterung kann durch Zugabe geeigneter saurer Katalysatoren mit einem pKₐ- Wert von kleiner gleich 5 oder durch Auskreisen des Reaktionswassers mit geeigneten Lösemitteln beschleunigt werden. Geeignete Carbonsäuren stellen Essigsäure, Propionsäure, Capronsäure, Caprylsäure, 2-Ethylhexansäure und Fettsäuren bzw. deren Anhydride, Methylester und Chloride dar.

Die Herstellung der erfindungsgemäßen Verbindungen erfolgt dann durch Quatemierung der tertiären Stickstoffatome mit einem geeigneten Alkylierungsmittel bei 50 bis 150 °C. Geeignete Alkylierungsmittel stellen Alkylhalogenide und Alkylsulfate dar, bevorzugt Methylenchlorid, Butylbromid und Dimethylsulfat.

### Beispiele:

### a) Allgemeine Vorschrift für die Herstellung alkoxylierter Amin- Ethercarbonsäuren

In einer Rührapparatur wurden 2 mol des entsprechenden alkoxylierten Amins bzw. 1 mol des entsprechenden alkoxylierten Diamins (nach OH-Zahl) unter Stickstoffspülung vorgelegt und auf 40 °C erwärmt. Dann wurden 650 g (4,8 mol) Natriumchloracetat für alkoxylierte Monoamine bzw. 488 g (3,6 mol) Natriumchloracetat für alkoxylierte Diamine eingetragen und die Reaktionsmischung auf 50 °C erwärmt. Nach jeweils 30 min wurden 192 g (4,8 mol) bzw. 144 g (3,6 mol) NaOH- Microprills in 6 Portionen so zugegeben, daß die Temperatur 55 °C nicht übersteigt. Es wurde 2 h bei 70 °C nachreagiert. Danach wurde 10 % Salzsäure zudosiert, bis ein pH < 3 erreicht wurde. Die Mischung wurde dann auf 95 °C erhitzt und in eine beheizbare Rührapparatur mit Bodenablaß überführt. Die Phasentrennung erfolgte nach 15 min bei 105-108 °C. Die wäßrige Uriterphase wurde verworfen. Bei Produkten; die sich nicht durch Erhitzen über den Trübungspunkt abtrennen lassen, wurde das Reaktionswasser destillativ entfernt und das dabei ausfallende Salz abfiltriert.

### Beispiel 1 (Cyclopentylamin + 2 EO-ECS)

Aus 370 g Cyclopentylamin + 2 EO (OH-Zahl: 606,0 mg KOH/g) wurden 600 g Cyclopentylamin + 2 EO-ECS mit SZ = 354,2 mg KOH/g (Ausbeute 95,0 % Umsatz) und bas.-N = 4,84 % erhalten.

### Beispiel 2 (Cyclopentylamin + 6 EO-ECS)

Aus 745 g Cyclopentylamin + 6 EO (OH-Zahl: 301,1 mg KOH/g) wurden 1017 g Cyclopentylamin + 6 EO-ECS mit SZ = 212,4 mg KOH/g (entspricht 92.5 % Umsatz) und bas.-N = 2,84 % erhalten.

### Beispiel 3 (Cyclohexylamin + 2 EO-ECS)

Aus 398 g Cyclohexylamin + 2 EO (OH-Zahl: 564,0 mg KOH/g) wurden 627 g Cyclohexylamin + 2 EO-ECS mit SZ = 341,6 mg KOH/g (entspricht 95.9 % Umsatz) und bas.-N = 4,50 % erhalten.

### Beispiel 4 (Cyclohexylamin + 6 EO-ECS)

Aus 725 g Cyclopentylamin + 6 EO (OH-Zahl: 309,6 mg KOH/g) wurden 975 g Cyclopentylamin + 6 EO-ECS mit SZ = 220,3 mg KOH/g (entspricht 93.9 % Umsatz) und bas.-N = 2,89 % erhalten.

### Beispiel 5 (n-Butylamin + 2 EO-ECS)

Aus 346 g n-Butylamin + 2 EO (OH-Zahl: 648,7 mg KOH/g) wurden 579 g n-Butylamin + 2 EO-ECS mit SZ = 377,1 mg KOH/g (entspricht 97.1 % Umsatz) und bas.-N = 4,62 % erhalten.

### Beispiel 6 (n-Butylamin + 6 EO-ECS)

Aus 699 g n-Butylamin + 6 EO (OH-Zahl: 321,1 mg KOH/g) wurden 970 g n-Butylamin + 6 EO-ECS mit SZ = 221,5 mg KOH/g (entspricht 91.9 % Umsatz) und bas.-N = 3,00 % erhalten.

### Beispiel 7 (n-Butylamin + 10 EO-ECS)

Aus 1032 g n-Butylamin + 10 EO (OH-Zahl: 217,5 mg KOH/g) wurden 1320 g n-Butylamin + 10 EO-ECS mit SZ = 148,7 mg KOH/g (entspricht 83.7 % Umsatz) und bas.-N = 1,89 % erhalten.

### Beispiel 8 (iso-Butylamin + 6 EO-ECS)

Aus 722 g iso-Butylamin + 6 EO (OH-Zahl: 310,9 mg KOH/g) wurden 995 g iso-Butylamin + 6 EO-ECS mit SZ = 219,2 mg KOH/g (entspricht 93.2 % Umsatz) und bas.-N = 3,01 % erhalten.

### Beispiel 9 (iso-Butylamin + 10 EO-ECS)

Aus 1120 g iso-Butylamin + 10 EO (OH-Zahl: 200,4 mg KOH/g) wurden 1384 g iso-Butylamin + 10 EO-ECS mit SZ = 135,6 mg KOH/g (entspricht 91.6 % Umsatz) und bas.-N = 2,08 % erhalten.

### Beispiel 10 (Caprylamin + 6 EO-ECS)

Aus 801 g Caprylamin + 6 EO (OH-Zahl: 280,1 mg KOH/g) wurden 1045 g Caprylamin + 6 EO-ECS mit SZ = 200,9 mg KOH/g (entspricht 92.5 % Umsatz) und bas.-N = 2,69 % erhalten.

### Beispiel 11 (Caprylamin + 10 EO-ECS)

Aus 1147 g Caprylamin + 10 EO (OH-Zahl: 195,7 mg KOH/g) wurden 1412 g Caprylamin + 10 EO-ECS mit SZ = 144,9 mg KOH/g (entspricht 89.0 % Umsatz) und bas.-N = 1,90 % erhalten.

### Beispiel 12 (Talgfett-propylendiamin + 10 EO-ECS)

Aus 768 g Talgfett-propylendiamin + 10 EO (OH-Zahl: 21.9,2 mg KOH/g) wurden 970 g Talgfett-propylendiamin + 10 EO-ECS mit SZ = 156,7 mg KOH/g (entspricht 87.7 % Umsatz) und bas.-N = 2,88 % erhatten:

### Beispiel 13 (Talgfett-propylendiamin + 25 EO-ECS)

Aus 1316 g Talgfett-propylendiamin + 25 EO (OH-Zahl: 127,9 mg KOH/g) wurden 1700 g Talgfett-propylendiamin + 25 EO-ECS mit SZ = 85,0 mg KOH/g (entspricht 84.0 % Umsatz) und bas.-N = 1,49 % erhalten.

### Beispiel 14 (Talgfett-propylendiamin + 30 EO-ECS)

Aus 1699 g Talgfett-propylendiamin + 30 EO (OH-Zahl: 99,1 mg KOH/g) wurden 2043 g Talgfett-propylendiamin + 30 EO-ECS mit SZ = 66,5 mg KOH/g (entspricht 80.9 % Umsatz) und bas.-N = 1,30 % erhalten.

### Beispiel 15 (Talgfett-propylendiamin + 35 EO-ECS)

Aus 1919 g Talgfett-propylendiamin + 35 EO (OH-Zahl: 87,7 mg KOH/g) wurden 2301 g Talgfett-propylendiamin + 35 EO-ECS mit SZ = 63,2 mg KOH/g (entspricht 85.5 % Umsatz) und bas.-N = 1,19 % erhalten.

### Beispiel 16 (Lauryl-propylendiamin + 10 EO-ECS)

Aus 673 g Lauryl-propylendiamin + 10 EO (OH-Zahl: 250,0 mg KOH/g) wurden 1071 g Lauryl-propylendiamin + 10 EO-ECS mit SZ = 149,2 mg KOH/g (entspricht 90.5 % Umsatz) und bas.-N = 2,54 % erhalten.

### Beispiel 17 (Lauryl-propylendiamin + 30 EO-ECS)

Aus 1639 g Lauryl-propylendiamin + 30 EO (OH-Zahl: 102,7 mg KOH/g) wurden 1964 g Lauryl-propylendiamin + 30 EO-ECS mit SZ = 82,3 mg KOH/g (entspricht 97.1 % Umsatz) und bas.-N = 1,40 % erhalten.

### b) Allgemeine Vorschrift für die Herstellung alkoxylierter Amin-Ethercarbonsäurealkylester

In einer Rührapparatur wurden 1 mol bzw. 0,5 mol (nach SZ) der entsprechenden alkoxylierten Alkylamin- bzw. Alkylendiamin- Ethercarbonsäure unter Stickstoffspülung vorgelegt und mit einem Überschuß (ca. 1,5 mol Equivalente) Alkohol versetzt. Nach Zugabe von 0,5 Gew. % FASCAT 4100 (Butylstannansäure) wurde die Mischung auf 100 °C bis 180 °C erhitzt, wobei das Reaktionswasser abdestillierte. Nach einer Reaktionszeit von 8 h bzw. Erreichen einer Säurezahl von SZ < 5 mg KOH/g wurde die Umsetzung beendet und überschüssiger Alkohol bzw. Restwasser destillativ im Vakuum entfernt.

### Beispiel 18 (Cyclopentylamin + 2 EO-2-ethylhexyl-ECS-ester)

Aus 317 g Cyclopentylamin + 2 EO-ECS und 391 g 2-Ethylhexanol wurden 521 g Cyclopentylamin + 2 EO-2-ethylhexyl-ECS-ester mit SZ = 2,8 mg KOH/g und VZ = 209,3 mg KOH/g (entspricht 98,7 % Umsatz) erhalten.

### Beispiel 19 (Cyclopentylamin + 6 EO-2-ethylhexyl-ECS-ester)

Aus 528 g Cyclopentylamin + 6 EO-ECS und 391 g 2-Ethylhexanol wurden 705 g Cyclopentylamin + 6 EO-2-ethylhexyl-ECS-ester mit SZ = 4,9 mg KOH/g und VZ = 154,1 mg KOH/g (entspricht 96,8 % Umsatz) erhalten.

### Beispiel 20 (Cyclohexylamin + 2 EO-2-ethylhexyl-ECS-ester)

Aus 329 g Cyclohexylamin + 2 EO-ECS und 391 g 2-Ethylhexanol wurden 536 g Cyclohexylamin + 2 EO-2-ethylhexyl-ECS-ester mit SZ = 1,8 mg KOH/g und VZ = 207,2 mg KOH/g (entspricht 99,1 % Umsatz) erhalten.

### Beispiel 21 (Cyclohexylamin + 6 EO-2-ethylhexyl-ECS-ester)

Aus 509 g Cyclopentylamin + 6 EO-ECS und 391 g 2-Ethylhexanol wurden 699 g Cyclopentylamin + 6 EO-2-ethylhexyl-ECS-ester mit SZ = 3,3 mg KOH/g und VZ = 153,4 mg KOH/g (entspricht 97,8 % Umsatz) erhalten.

### Beispiel 22 (n-Butylamin + 2 EO-2-ethylhexyl-ECS-ester)

Aus 298 g n-Butylamin + 2 EO-ECS und 391 g 2-Ethylhexanol wurden 503 g n-Butylamin + 2 EO-2-ethylhexyl-ECS-ester mit SZ = 2,4 mg KOH/g und VZ = 219,5 mg KOH/g (entspricht 98,9 % Umsatz) erhalten.

### Beispiel 23 (n-Butylamin + 6 EO-2-ethylhexyl-ECS-ester)

Aus 507 g n-Butylamin + 6 EO-ECS und 391 g 2-Ethylhexanol wurden 707 g n-Butylamin + 6 EO-2-ethylhexyl-ECS-ester mit SZ = 4,1 mg KOH/g und VZ = 158,1 mg KOH/g (entspricht 97,4 % Umsatz) erhalten.

### Beispiel 24 (n-Butylamin + 10 EO-dodecyl-ECS-ester)

Aus 1032 g n-Butylamin + 10 EO-ECS und 559 g Laurylalkohol wurden 1320 g n-Butylamin + 10 EO-dodecyl-ECS-ester mit SZ = 8,7 mg KOH/g und VZ = 124,3 mg KOH/g (entspricht 92,9 % Umsatz) erhalten.

### Beispiel 25 (iso-Butylamin + 6 EO-2-ethylhexyl-ECS-ester)

Aus 512 g iso-Butylamin + 6 EO-ECS und 391 g 2-Ethylhexanol wurden 683 g iso-Butylamin + 6 EO-2-ethylhexyl-ECS-ester mit SZ = 5,1 mg KOH/g und VZ = 152,3 mg KOH/g (entspricht 96,7 % Umsatz) erhalten.

### Beispiel 26 (iso-Butylamin + 10 EO-dodecyl-ECS-ester)

Aus 1120 g iso-Butylamin + 10 EO-ECS und 559 g Laurylalkohol wurden 1384 g iso-Butylamin + 10 EO-dodecyl-ECS-ester mit SZ = 5,6 mg KOH/g und VZ = 115,4 mg KOH/g (entspricht 95,2 % Umsatz) erhalten.

### Beispiel 27 (Caprylamin + 6 EO-2-ethylhexyl-ECS-ester)

Aus 559 g Caprylamin + 6 EO-ECS und 391 g 2-Ethylhexanol 738 g Caprylamin + 6 EO-2-ethylhexyl-ECS-ester mit SZ = 3,3 mg KOH/g und VZ = 147,0 mg KOH/g (entspricht 97,8 % Umsatz) erhalten.

### Beispiel 28 (Caprylamin + 10 EO-2-ethylhexyl-ECS-ester)

Aus 774 g Caprylamin + 10 EO-ECS und 391 g 2-Ethylhexanol wurden 999 g Caprylamin + 10 EO-2-ethylhexyl-ECS-ester mit SZ = 4,8 mg KOH/g und VZ = 114,1 mg KOH/g (entspricht 95,8 % Umsatz) erhalten.

### Beispiel 29 (Talgfett-propylendiamin + 10 EO-2-ethylhexyl-ECS-ester)

Aus 537 g Talgfett-propylendiamin + 10 EO-ECS und 293 g 2-Ethylhexanol wurden 688 g Talgfett-propylendiamin + 10 EO-2-ethylhexyl-ECS-ester mit SZ = 4,7 mg KOH/g und VZ = 121,3 mg KOH/g (entspricht 96,1 % Umsatz) erhalten.

### Beispiel 30 (Talgfett-propylendiamin + 25 EO-ethylhexyl-ECS-ester)

Aus 990 g Talgfett-propylendiamin + 25 EO-ECS und 293 g 2-Ethylhexanol wurden 1068 g Talgfett-propylendiamin + 25 EO-2-ethylhexyl-ECS-ester mit SZ = 6,7 mg KOH/g und VZ = 74,6 mg KOH/g (entspricht 91,0 % Umsatz) erhalten.

### Beispiel 31 (Talgfett-propylendiamin + 30 EO-ethylhexyl-ECS-ester)

Aus 1266 g Talgfett-propylendiamin + 30 EO-ECS und 293 g 2-Ethylhexanol wurden 1374 g Talgfett-propylendiamin + 30 EO-2-ethylhexyl-ECS-ester mit SZ = 3,5 mg KOH/g und VZ = 61,7 mg KOH/g (entspricht 94,3 % Umsatz) erhalten.

### Beispiel 32 (Talgfett-propylendiamin + 35 EO-dodecyl-ECS-ester)

Aus 1332 g Talgfett-propylendiamin + 35 EO-ECS und 419 g Laurylalkohol wurden 1523 g Talgfett-propylendiamin + 35 EO-2-dodecyl-ECS-ester mit SZ = 4,9 mg KOH/g und VZ = 54,2 mg KOH/g (entspricht 90,9 % Umsatz) erhalten.

### Beispiel 33 (Lauryl-propylendiamin + 10 EO-2-ethylhexyl-ECS-ester)

Aus 564 g Lauryl-propylendiamin + 10 EO-ECS und 293 g 2-Ethylhexanol wurden 703 g Lauryl-propylendiamin + 10 EO-2-ethylhexyl-ECS-ester mit SZ = 3,6 mg KOH/g und VZ = 117,9 mg KOH/g (entspricht 96,9 % Umsatz) erhalten.

### Beispiel 34 (Lauryl-propylendiamin + 30 EO-2-dodecyl-ECS-ester)

Aus 1023 g Lauryl-propylendiamin + 30 EO-ECS und 419 g Laurylalkohol wurden 1213 g Lauryl-propylendiamin + 30 EO-2-dodecyl-ECS-ester mit SZ = 6,0 mg KOH/g und VZ = 66,8 mg KOH/g (entspricht 91,0 % Umsatz) erhalten.

### c) Allgemeine Vorschrift für die Herstellung alkoxylierter Amin-Carbonsäureester durch Umsetzung mit Carbonsäuren

In einer Rührapparatur wurden 1 mol bzw. 0,5 mol (nach OH-Zahl) des entsprechenden alkoxylierten Alkylamins bzw. Alkylendiamins unter Stickstoffspülung vorgelegt und mit 1 mol Equivalenten der entsprechenden Carbonsäure versetzt. Nach Zugabe von 0,5 Gew. % FASCAT 4100 (Butylstannansäure) wurde die Mischung auf 100 bis 200°C erhitzt, wobei das Reaktionswasser abdestillierte. Nach einer Reaktionszeit von 8 h bzw. Erreichen einer Säurezahl von SZ < 10 mg KOH/g wurde die Umsetzung beendet und Restwasser destillativ im Vakuum entfernt.

### d) Allgemeine Vorschrift für die Herstellung alkoxylierter Amin-Carbonsäureester durch Umsetzung mit Carbonsäureanhydriden

In einer Rührapparatur wurden 1 mol bzw. 0,5 mol (nach OH-Zahl) des entsprechenden alkoxylierten Alkylamins bzw. Alkylendiamins unter Stickstoffspülung vorgelegt und mit 1 mol Equivalent des entsprechenden Carbonsäureanhydrids vesetzt. Die Mischung wurde auf 100 bis 150°C erhitzt. Nach einer Reaktionszeit von 8 h bei dieser Reaktionstemperatur wurde die freigesetzte Carbonsäure abdestilliert.

### Beispiel 35 (n-Butylamin + 2 EO-essigsäureester)

Aus 173 g n-Butylamin + 2 EO (OH-Zahl: 648,7 mg KOH/g) und 204 g Essigsäureanhydrid wurden 262 g n-Butylamin + 2 EO-essigsäureester mit SZ = 0,4 mg KOH/g und VZ = 440,7 mg KOH/g erhalten.

### Beispiel 36 (n-Butylamin + 6 EO-essigsäureester)

Aus 349 g n-Butylamin + 6 EO (OH-Zahl: 321,1 mg KOH/g) und 204 g Essigsäureanhydrid wurden 434 g n-Butylamin + 6 EO-essigsäureester mit SZ = 0,1 mg KOH/g und VZ = 260,2 mg KOH/g erhalten.

### Beispiel 37 (n-Butylamin + 6 EO-propionsäureester)

Aus 349 g n-Butylamin + 6 EO (OH-Zahl: 321,1 mg KOH/g) und 260 g Propionsäureanhydrid wurden 465 g n-Butylamin + 6 EO-propionsäureester mit SZ = 0,7 mg KOH/g und VZ = 244,9 mg KOH/g erhalten.

### Beispiel 38 (n-Butylamin + 6 EO-2-ethylhexansäureester)

Aus 349 g n-Butylamin + 6 EO-ECS (OH-Zahl: 321,1 mg KOH/g) und 288 g 2-Ethylhexansäure wurden 594 g n-Butylamin + 6 EO-2-ethylhexansäureester mit SZ = 6,4 mg KOH/g und VZ = 191,8 mg KOH/g erhalten.

### Beispiel 39 (Caprylamin + 6 EO-essigsäureester)

Aus 401 g Caprylamin + 6 EO (OH-Zahl: 280,1 mg KOH/g) und 204 g Essigsäureanhydrid wurden 484 g Caprylamin + 6 EO-essigsäureester mit SZ = 0,2 mg KOH/g und VZ = 231,5 mg KOH/g erhalten.

### Beispiel 40 (Caprylamin + 6 EO-propionsäureester)

Aus 401 g Caprylamin + 6 EO (OH-Zahl: 280,1 mg KOH/g) und 260 g Propionsäureanhydrid wurden 517 g Caprylamin + 6 EO-propionsäureester mit SZ = 0,4 mg KOH/g und VZ = 220,8 mg KOH/g erhalten.

### Beispiel 41 (Caprylamin + 6 EO-2-ethylhexansäureester)

Aus 401 g Caprylamin + 6 EO (OH-Zahl: 280,1 mg KOH/g) und 288 g 2-Ethylhexansäure wurden 643 g Caprylamin + 6 EO-2-ethylhexansäureester mit SZ = 8,1 mg KOH/g und VZ = 179,6 mg KOH/g erhalten.

### Beispiel 42 (Taigfett-propylendiamin + 25 EO-propionsäureester)

Aus 658 g Talgfett-propylendiamin + 25 EO (OH-Zahl: 127,9 mg KOH/g) und 195 g Propionsäureanhydrid wurden 750 g Talgfett-propylendiamin + 25 EO-propionsäureester mit SZ = 0,7 mg KOH/g und VZ = 114,3 mg KOH/g erhalten.

### Beispiel 43 (Talgfett-propylendiamin + 25 EO-2-ethylhexansäureester)

Aus 658 g Talgfett-propylendiamin + 25 EO (OH-Zahl: 127,9 mg KOH/g) und 216 g 2-Ethylhexansäure wurden 859 g Talgfett-propylendiamin + 25 EO-2-ethylhexansäureester mit SZ = 8,6 mg KOH/g und VZ = 107,6 mg KOH/g erhalten.

### Beispiel 44 (Talgfett-propylendiamin + 25 EO-cocosfettsäureester)

Aus 658 g Talgfett-propylendiamin + 25 EO (OH-Zahl: 127,9 mg KOH/g) und 310 g Cocosfettsäure (SZ = 271,3 mg KOH/g) wurden 951 g Talgfett-propylendiamin + 25 EO- cocosfettsäureester mit SZ = 4,5 mg OH/g und VZ = 93,9 mg KOH/g erhalten.

### Beispiel 45 (Lauryl-propylendiamin + 30 EO-cocosfettsäureester)

Aus 820 g Lauryl-propylendiamin + 30 EO (OH-Zahl: 102,7 mg KOH/g) und 310 g Cocosfettsäure (SZ = 271,3 mg KOH/g) wurden 1107 g Lauryl-propylendiamin + 30 EO-cocosfettsäureester mit SZ = 3,6 mg KOH/g und VZ = 79,9 mg KOH/g erhalten.

### e) Allgemeine Vorschrift für die Quaternierung der alkoxylierten Amin-Ethercarbonsäurealkylester bzw. der alkoxylierten Amin- Carbonsäureester

In einer Rührapparatur wurden 0,5 mol (nach VZ-Zahl) des entsprechenden alkoxylierten Amin- Ethercarbonsäurealkylesters bzw. des alkoxylierten Amin-Carbonsäureesters unter Stickstoffspülung vorgelegt und auf 60°C erwärmt. Dazu wurden 0,4 mol Dimethylsulfat so zugetropft, daß die Reaktionstemperatur 80-90°C nicht übersteigt. Die Reaktionsmischung wurde anschließend 3 h bei 90°C nachgerührt. Nach dieser Vorschrift wurden die Verbindungen, beschrieben durch die Beispiele 18 bis 45 quaternisiert (Beispiele 46 bis 73, wie in Tabelle 1 aufgeführt).

### Beispiel 74

Polyvinylcaprolactam mit MW 5000 g/mol werden im Verhältnis 1:1 mit dem Quat, beschrieben durch Beispiel 51, vermischt und in Butyldiglykol eingestellt.

### Beispiel 75

Polyvinylcaprolactam mit MW 5000 g/mol werden im Verhältnis 1:1 mit dem Quat, beschrieben durch Beispiel 66, vermischt und in Butyldiglykol eingestellt.

### Wirksamkeit der erfindungsgemäßen Verbindungen als Gashydratinhibitoren

Zur Untersuchung der inhibierenden Wirkung der erfindungsgemäßen Verbindungen wurde ein Stahl-Rührautoklav mit Temperatursteuerung, Druck- und Drehmomentaufnehmer mit 450 ml Innenvolumen benutzt. Für Untersuchungen zur kinetischen Inhibierung wurde der Autoklav mit destilliertem Wasser und Gas im Volumenverhältnis 20: 80 gefüllt, für Untersuchungen der Agglomeratinhibierung wurde zusätzlich Kondensat zugegeben. Abschließend wurde bei unterschiedlichen Drücken Erdgas aufgepresst.

Ausgehend von einer Anfangstemperatur von 17,5°C wurde innerhalb von 2 h auf 2°C abgekühlt, dann 18 h bei 2°C gerührt und innerhalb von 2 h wieder auf 17,5°C aufgeheizt. Dabei wird zunächst eine Druckabnahme gemäß der thermischen Kompression des Gases beobachtet. Tritt während der Unterkühlzeit die Bildung von Gashydratkeimen auf, so verringert sich der gemessene Druck, wobei ein Anstieg des gemessenen Drehmoments und ein leichter Anstieg der Temperatur zu beobachten ist. Weiteres Wachstum und zunehmende Agglomeration der Hydratkeime führen ohne Inhibitor schnell zu einem weiteren Anstieg des Drehmomentes. Beim Erwärmen des Gemisches zerfallen die Gashydrate, so dass man den Anfangszustand der Versuchsreihe erreicht.

Als Maß für die inhibierende Wirkung der erfindungsgemäßen Verbindungen wird die Zeit vom Erreichen der Minimaltemperatur von 2°C bis zur ersten Gasaufnahme (T_{ind}) bzw. die Zeit bis zum Anstieg des Drehmomentes (T_{agg}) benutzt. Lange Induktionszeiten bzw. Agglomerationszeiten weisen auf eine Wirkung als kinetischer Inhibitor hin. Das im Autoklaven gemessene Drehmoment dient dagegen als Größe für die Agglomeration der Hydratkristalle. Der gemessene Druckverlust (Δ p) lässt einen direkten Schluss auf die Menge gebildeter Hydratkristalle zu. Bei einem guten Antiagglomerat ist das Drehmoment, das sich nach Bildung von Gashydraten aufbaut, gegenüber dem Blindwert deutlich verringert. Im Idealfall bilden sich schneeartige, feine Hydratkristalle in der Kondensatphase, die sich nicht zusammenlagern und somit nicht zum Verstopfen der zum Gastransport und zur Gasförderung dienenden Installationen führen.

### Testergebnisse

### Zusammensetzung des verwendeten Erdgases:

- Gas 1:: Methan 79,3 %, Ethan 10,8 %, Propan 4,8 %, Butan 1,9 %, Kohlendioxid 1,4 %, Stickstoff 1,8 %. Unterkühlung unter die Gleichgewichtstemperatur der Hydratbildung bei 50 bar: 12°C.
- Gas 2:: Methan 92,1 %, Ethan 3,5 %, Propan 0,8 %, Butan 0,7 %, Kohlendioxid 0,6 %, Stickstoff 2,3 %. Unterkühlung unter die Gleichgewichtstemperatur der Hydratbildung bei 50 bar: 7°C, Unterkühlung bei 100 bar: 12°C.

Um die Wirkung als Agglomeratinhibitoren zu prüfen, wurde im oben verwendeten Testautoklaven Wasser und Testbenzin vorgelegt (20 % des Volumens im Verhältnis 1:2) und bezogen auf die Wasserphase 5000 ppm des jeweiligen Additivs zugegeben. Bei einem Autoklavendruck von 90 bar unter Verwendung von Gas 1 und einer Rührgeschwindigkeit von 5000 U/min wurde die Temperatur von anfangs 17,5°C innerhalb 2 Stunden auf 2°C abgekühlt, dann 25 Stunden bei 2°C gerührt und wieder aufgewärmt. Dabei wurde der Druckverlust durch die Hydratbildung und das dabei auftretende Drehmoment am Rührer gemessen, das ein Maß für die Agglomeration der Gashydrate ist.

**Tabelle 1:**

| (Test als Antiagglomerant) | | | | |
|---|---|---|---|---|
| Beispiel | Quat aus | Druckverlust Δ p (bar) | Temperaturanstieg Δ T (K) | Drehmoment Mₘₐₓ (Ncm) |
| Blindwert | | > 40 | > 8 | 15,9 |
| 46 | Beispiel 18 | 15,1 | 0,3 | 0,3 |
| 47 | Beispiel 19 | 23,1 | 2,2 | 6,3 |
| 48 | Beispiel 20 | 15,3 | 0,7 | 0,4 |
| 49 | Beispiel 21 | 19,9 | 1,9 | 5,7 |
| 50 | Beispiel 22 | 10,1 | 0,1 | 0,2 |
| 51 | Beispiel 23 | 12,3 | 0,2 | 0,2 |
| 52 | Beispiel 24 | 16,8 | 0,8 | 0,9 |
| 53 | Beispiel 25 | 13,4 | 0,2 | 0,3 |
| 54 | Beispiel 26 | 10,9 | 0,2 | 0,3 |
| 55 | Beispiel 27 | 17,4 | 1,9 | 5,8 |
| 56 | Beispiel 28 | 16,6 | 1,0 | 0,9 |
| 57 | Beispiel 29 | 28,5 | 3,2 | 8,8 |
| 58 | Beispiel 30 | 22,1 | 2,5 | 8,3 |
| 59 | Beispiel 31 | 15,8 | 0,8 | 0,5 |
| 60 | Beispiel 32 | 20,6 | 2,0 | 4,9 |
| 61 | Beispiel 33 | 16,2 | 1,1 | 0,9 |
| 62 | Beispiel 34 | 26,8 | 5,1 | 9,2 |
| 63 | Beispiel 35 | 10,3 | 0,1 | 0,1 |
| 64 | Beispiel 36 | 12,8 | 0,4 | 0,5 |
| 65 | Beispiel 37 | 11,6 | 0,4 | 0,4 |
| 66 | Beispiel 38 | 9,4 | 0,0 | 0,0 |
| 67 | Beispiel 39 | 23,0 | 3,5 | 2,4 |
| 68 | Beispiel 40 | 19,0 | 2,5 | 1,4 |
| 69 | Beispiel 41 | 17,0 | 1,5 | 1,2 |
| 70 | Beispiel 42 | 27,1 | 5,9 | 4,8 |
| 71 | Beispiel 43 | 26,8 | 5,8 | 4,4 |
| 72 | Beispiel 44 | 14,8 | 0,8 | 0,9 |
| 73 | Beispiel 45 | 14,5 | 0,5 | 1,0 |
| Vergleich | | 21,5 | 1,0 | 1,5 |
| Vergleich | | 15,0 | 1,0 | 1,2 |

Als Vergleichssubstanz wurden zwei kommerziell erhältliche Antiägglomerant-Inhibitoren aus Basis Tetrabutylammoniumbromid herangezogen.

Wie aus diesen Beispielen zu ersehen ist, waren die gemessenen Drehmomente im Vergleich zum Blindwert trotz heftiger Hydratbildung stark reduziert. Dies spricht für eine deutliche agglomeratinhibierende Wirkung der erfindungsgemäßen Produkte. Es ist offensichtlich, dass besonders bei ausgewogener HL- Balance exzellente Resultate erzielt werden.

Um die Wirkung als Additive für kinetische Inhibitoren zu prüfen, wurde im oben verwendeten Testautoklaven 5000 ppm des jeweiligen Additivs bezogen auf die Wasserphase zugegeben und bei unterschiedlichen Drücken unter Verwendung der Gase 1 bzw. 2 abgekühlt. Nach Erreichen der Minimaltemperatur von 2°C wurde die Zeit bis zur ersten Gasaufnahme (T_{ind}) aufgezeichnet. Der gemessene Druckverlust (Δ p) und der Temperaturanstieg Δ T (K) erlaubt den direkten Schluss auf die Menge gebildeter Hydratkristalle.

**Tabelle 2:**

| (Test als kinetische Inhibitoren) | | | | | | |
|---|---|---|---|---|---|---|
| Beispiel | Inhibitor | Gas | Druck p (bar) | T_{Ind} | Druckverlust Δ p (bar) | Temperaturanstieg Δ T (K) |
| Blindwert | - | 1 | 50 | 0 | > 40 | > 1,5 |
| Blindwert | - | 2 | 100 | 0 | > 40 | > 1,5 |
| 76 | Beispiel 74 | 1 | 50 | 18,5 h | 0 | 0,0 |
| 77 | Beispiel 74 | 2 | 100 | < 5 min | 6,8 | 0,2 |
| 78 | Beispiel 75 | 1 | 50 | 9,0 h | 9,7 | 0;4 |
| 79 | Beispiel 75 | 2 | 50 | 6,5 h | 11,2 | 0,3 |
| 80 | Beispiel 75 | 2 | 100 | 1 h | 10,5 | 0,3 |
| Vergleich | PVCap | 1 | 50 | < 5 min | 10 | 0,4 |
| Vergleich | PVCap | 2 | 100 | < 5 min | 6 | 0,1 |

Als Vergleichssubstanz wurde eine Lösung von Polyvinylcaprolactam (PVCap) in Butylglykol herangezogen, Molekulargewicht 5000 g/mol.

Wie aus den obigen Testresultaten zu erkennen ist, wirken die erfindungsgemäßen Produkte als synergistische Komponente von kinetische Hydratinhibitoren und zeigen eine deutliche Verbesserung gegenüber dem Stand der Technik. Sie können daher zur Steigerung (Synergieeffekt) der Leistungsfähigkeit von Inhibitoren des Standes der Technik verwendet werden.

Die korrosionsinhibierenden Eigenschaften der erfindungsgemäßen Verbindungen wurden im Shell-wheel-test nachgewiesen. Coupons aus C-Stahl (DIN 1.1203 mit 15 cm² Oberfläche) wurden in eine Salzwasser/Petroleum-Mischung (9:1,5 %ige NaCl- Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

**Tabelle 3:**

| (SHELL-Wheel-Test) | | |
|---|---|---|
| Beispiel | Korrosionsinhibitor | Schutz % |
| Vergleich | | 35-40 |
| 81 | Beispiel 66 | 86-90 |
| 82 | Beispiel 69 | 85-88 |
| 83 | Beispiel 72 | 84-90 |

Die Produkte wurden außerdem im LPR-Test (Testbedingungen analog ASTM D 2776) geprüft.

**Tabelle 4:**

| (LPR-Test) | | | | |
|---|---|---|---|---|
| Beispiel | Korrosionsinhibitor | Schutz nach [%] | | |
| | | 10 min | 30 min | 60 min |
| Vergleich | | 53,9 | 61,2 | 73,7 |
| 84 | Beispiel 66 | 67,7 | 75,6 | 79,0 |
| 85 | Beispiel 69 | 78,0 | 85,7 | 87,9 |
| 86 | Beispiel 72 | 53,9 | 67,1 | 78,6 |

Als Vergleichssubstanz wurde bei beiden Test ein Rückstandsamin - Quat auf Basis Dicocosalkyl-dimethylammoniumchlorid (Korrosionsinhibitor des Standes der Technik) verwendet.

Wie aus den obigen Testresultaten zu erkennen ist, zeigen die erfindungsgemäßen Gashydratinhibitoren korrosionsinhibierende Eigenschaften und stellen somit eine deutliche Verbesserung gegenüber dem Stand der Technik dar. Somit kann bei Verwendung der Verbindungen als Gashydratinhibitor gegebenenfalls auf eine zusätzliche Additivierung mit einem Korrosionsinhibitor verzichtet werden. Ein aufwendiges Formulieren unter Berücksichtigung der Verträglichkeit von Gashydratinhibitor und Korrosionsschutzkomponente kann für den Anwender entfallen.

## Patentansprüche

1. Verwendung von Verbindungen der Formel 1 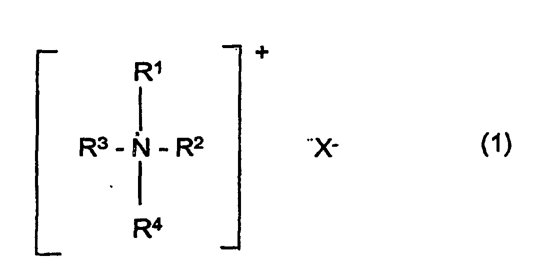 worin
R¹, R² unabhängig voneinander Reste der Formeln
-(B)-(O-A)ₙ-O-CO-R⁵ (2)
oder
-(A-O)ₙ-(C)-CO-O-R⁵ (3)
R³ C₁- bis C₃₀-Alkyl oder C₂- bis C₃₀-Alkenyl
R⁴ einen gegebenenfalls Heteroatome enthaltenden organischen Rest mit 1 bis 100 Kohlenstoffatomen
R⁵ C₁- bis C₃₀-Alkyl oder C₂- bis C₃₀-Alkenyl
n eine Zahl von 1 bis 20
A eine C₂- bis C₄-Alkylengruppe,
B eine C₁- bis C₁₀-Alkylengruppe,
C eine C₁- bis C₆-Alkylengruppe und
X ein Anion
bedeuten, als Gashydratinhibitoren.

2. Verwendung gemäß Anspruch 1, worin A für eine Ethylen- oder Propylengruppe steht.

3. Verwendung gemäß Anspruch 1 und/oder 2, worin B für eine C₂- bis C₄-Alkylengruppe steht.

4. Verwendung gemäß einem oder mehreren der Ansprüche 1 bis 3, worin C für eine C₂- bis C₄-Alkylengruppe steht.

5. Verwendung gemäß einem oder mehreren der Ansprüche 1 bis 4, worin n für eine Zahl zwischen 2 und 6 steht.

6. Verwendung gemäß einem oder mehreren der Ansprüche 1 bis 5, worin R⁵ für eine Alkyl- oder Alkenylgruppe mit 2 bis 24 Kohlenstoffatomen steht.

7. Verwendung gemäß einem oder mehreren der Ansprüche 1 bis 6, worin R³ für eine Alkyl- oder Alkenylgruppe von 2 bis 12 Kohlenstoffatomen steht.

8. Verwendung gemäß einem oder mehreren der Ansprüche 1 bis 7, worin R⁴ einem Rest der Formel (4) entspricht, 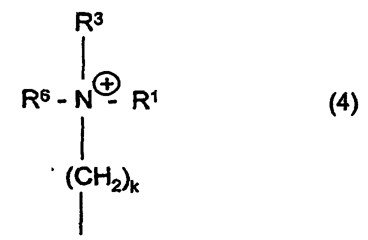 in dem R⁶ einen Rest der Formeln
-(B)-(O-A)ₙ-O-CO-R⁵ (2)
oder
-(A-O)ₙ-(C)-CO-O-R⁵ (3)
oder C₁- bis C₃₀-Alkyl oder C₂- bis C₃₀-Alkenyl und k 2 oder 3 bedeutet.

9. Verwendung gemäß einem oder mehreren der Ansprüche 1 bis 8, worin Verbindungen der Formeln (5) bis (8) 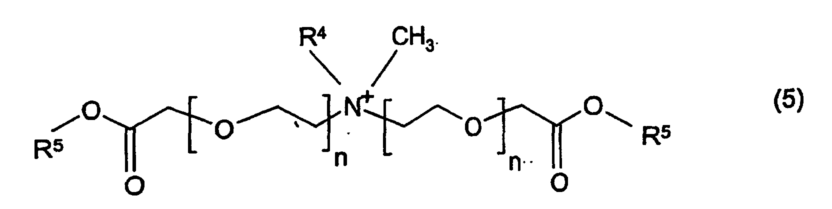 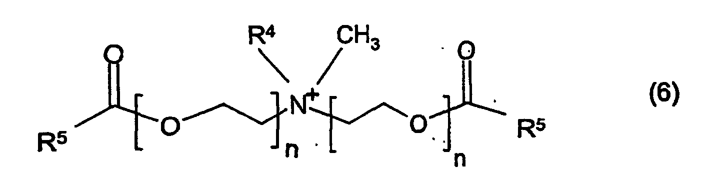 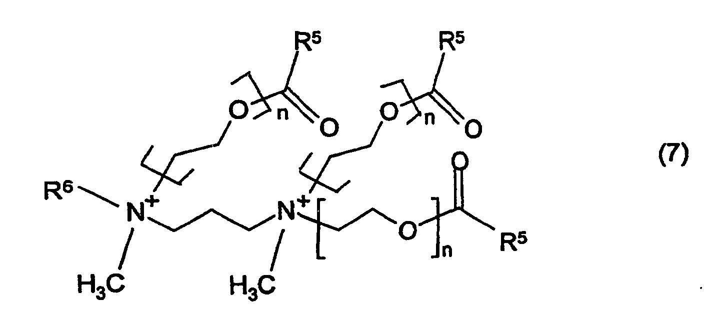 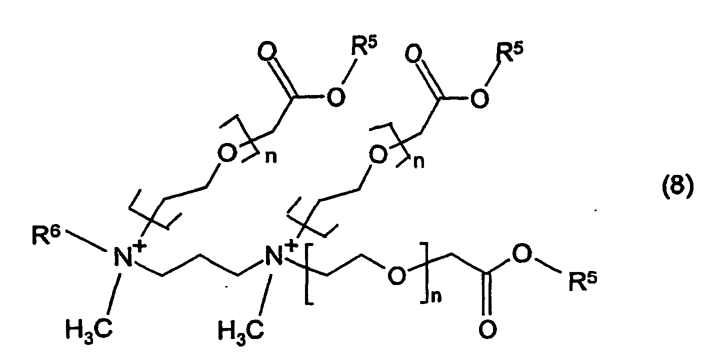 zur Anwendung kommen.

10. Verbindungen der Formel (1), wobei jedoch solche Verbindungen ausgeschlossen sind, in denen R⁴ kein Heteroatom enthält und R¹ und R² gleichzeitig die in Formel (2) angegebene Bedeutung aufweisen.

## Claims

1. The use of compounds of the formula 1 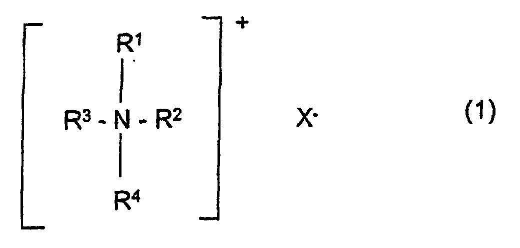 where
R¹, R² are each independently radicals of the formulae
-(B)-(O-A)ₙ-O-CO-R⁵ (2)
or
-(A-O)ₙ-(C)-CO-O-R⁵ (3)
R³ is C₁- to C₃₀-alkyl or C₂- to C₃₀-alkenyl,
R⁴ is an organic radical which optionally contains heteroatoms and has from 1 to 100 carbon atoms,
R⁵ is C₁- to C₃₀-alkyl or C₂- to C₃₀-alkenyl,
n is a number from 1 to 20,
A is a C₂- to C₄-alkylene group,
B is a C₁- to C₁₀-alkylene group,
C is a C₁- to C₆-alkylene group and
X is an anion
as gas hydrate inhibitors.

2. The use as claimed in claim 1, where A is an ethylene or propylene group.

3. The use as claimed in claim 1 and/or 2, where B is a C₂- to C₄-alkylene group.

4. The use as claimed in one or more of claims 1 to 3, where C is a C₂- to C₄-alkylene group.

5. The use as claimed in one or more of claims 1 to 4, where n is a number in the range from 2 to 6.

6. The use as claimed in one or more of claims 1 to 5, where R⁵ is an alkyl or alkenyl group having from 2 to 24 carbon atoms.

7. The use as claimed in one or more of claims 1 to 6, where R³ is an alkyl or alkenyl group having from 2 to 12 carbon atoms.

8. The use as claimed in one or more of claims 1 to 7, where R⁴ is a radical of the formula (4) 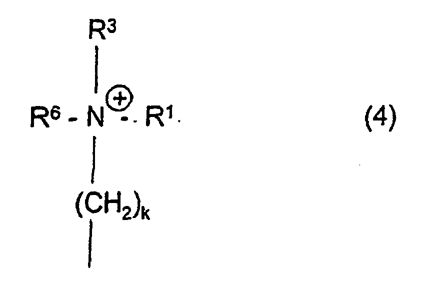 in which R⁶ is a radical of the formulae
-(B)-(O-A)ₙ-O-CO-R⁵ (2)
or
-(A-O)ₙ-(C)-CO-O-R⁵ (3)
or C₁- to C₃₀-alkyl or C₂- to C₃₀-alkenyl and k is 2 or 3.

9. The use as claimed in one or more of claims 1 to 8, where compounds of the formulae (5) to (8) 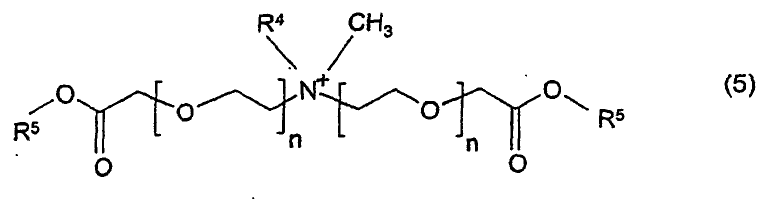 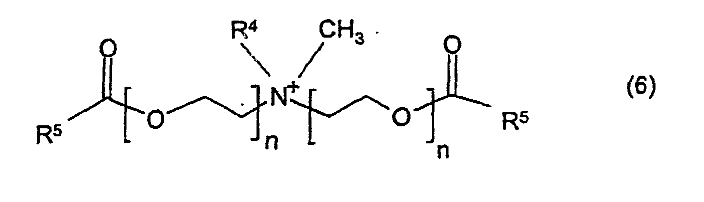 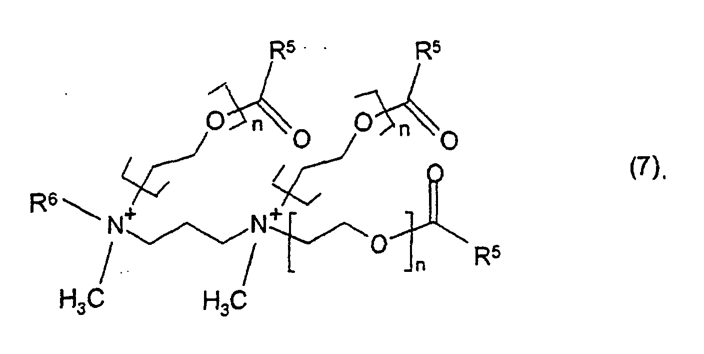 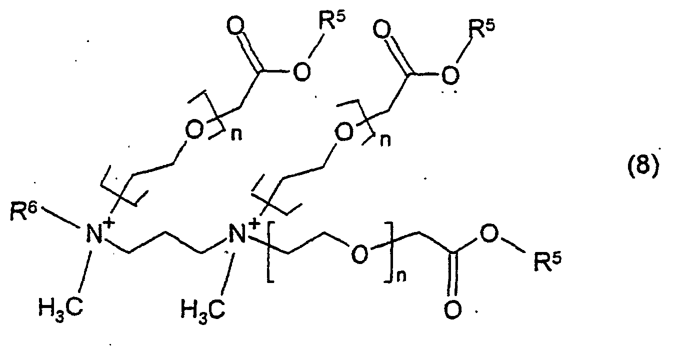 are used.

10. A compound of the formula (I), although excluding those compounds in which R⁴ contains no heteroatom and R¹ and R² are at the same time as defined in formula (2).

## Revendications

1. Utilisation de composés de formule 1 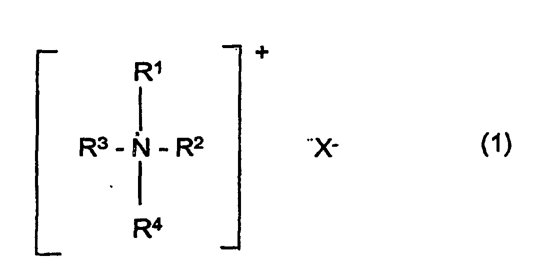 dans laquelle
R¹, R² représentent, indépendamment l'un de l'autre, des groupes de formules
-(B)-(O-A)ₙ-O-CO-R⁵ (2)
ou
-(A-O)ₙ-(C)-CO-O-R⁵ (3)
R³ représente un groupe alkyle en C₁ à C₃₀ ou alcényle en C₂ à C₃₀,
R⁴ représente un groupe organique possédant 1 à 100 atomes de carbone et contenant éventuellement des hétéroatomes,
R⁵ représente un groupe alkyle en C₁ à C₃₀ ou alcényle en C₂ à C₃₀,
n représente un nombre de 1 à 20,
A représente un groupe alkylène en C₂ à C₄,
B représente un groupe alkylène en C₁ à C₁₀,
C représente un groupe alkylène en C₁ à C₆ et
X est un anion,
en tant qu'inhibiteurs de formation d'hydrates de gaz.

2. Utilisation selon la revendication 1, dans laquelle A représente un groupe éthylène ou propylène.

3. Utilisation selon la revendication 1 et/ou 2, dans laquelle B représente un groupe alkylène en C₂ à C₄.

4. Utilisation selon l'une ou plusieurs des revendications 1 à 3, dans laquelle C représente un groupe alkylène en C₂ à C₄.

5. Utilisation selon l'une ou plusieurs des revendications 1 à 4, dans laquelle n représente un nombre compris entre 2 et 6.

6. Utilisation selon l'une ou plusieurs des revendications 1 à 5, dans laquelle R⁵ représente un groupe alkyle ou alcényle possédant 2 à 24 atomes de carbone.

7. Utilisation selon l'une ou plusieurs des revendications 1 à 6, dans laquelle R³ représente un groupe alkyle ou alcényle possédant 2 à 12 atomes de carbone.

8. Utilisation selon l'une ou plusieurs des revendications 1 à 7, dans laquelle R⁴ représente un groupe de formule (4) 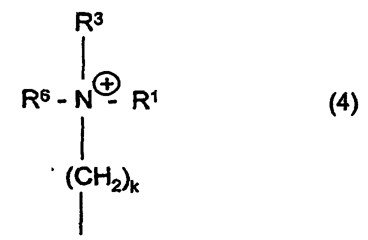 dans laquelle R⁶ représente un groupe de formules
-(B)-(O-A)ₙ-O-CO-R⁵ (2)
ou
-(A-O)ₙ-(C)-CO-O-R⁵ (3)
ou un groupe alkyle en C₁ à C₃₀ ou alcényle en C₂ à C₃₀ et k vaut 2 ou 3.

9. Utilisation selon l'une ou plusieurs des revendications 1 à 8, dans laquelle on met en oeuvre des composés de formules (5) à (8)

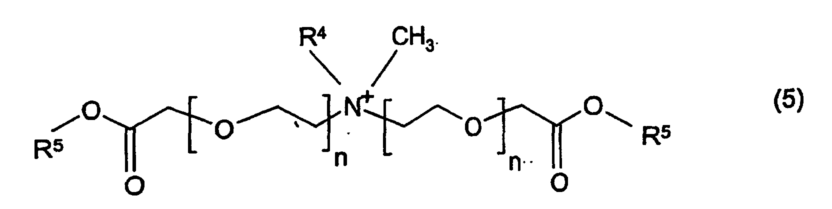

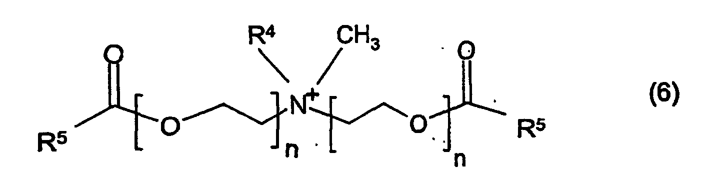

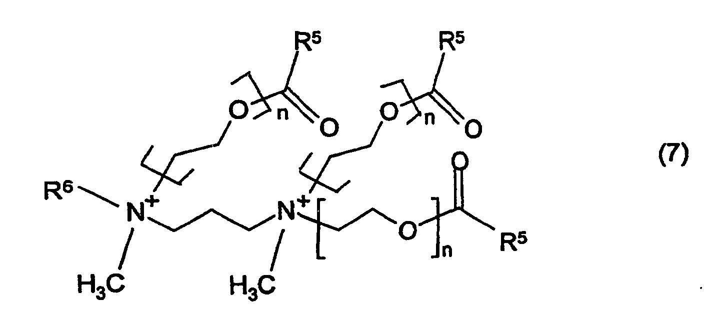

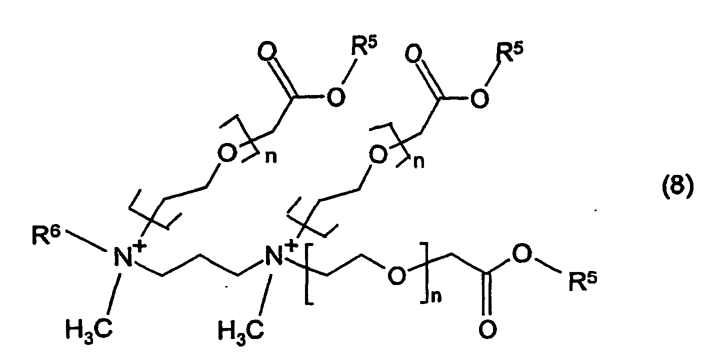

10. Composés de formule (1), desquels sont exclus les composés dans lesquels R⁴ ne contient pas d'hétéroatome et R¹ et R² ont en même temps la signification indiquée dans la formule (2).
